(19) Europäisches Patentamt
European Patent Office
Office européen des brevets

(11) Numéro de publication: **0 267 111**
**A1**

(12) # DEMANDE DE BREVET EUROPEEN

(21) Numéro de dépôt: **87402463.1**

(22) Date de dépôt: **02.11.87**

(51) Int. Cl.[4]: **C 07 D 471/04**
A 61 K 31/435
//(C07D471/04,235:00,221:00)

(30) Priorité: **07.11.86 FR 8615533**

(43) Date de publication de la demande:
**11.05.88 Bulletin 88/19**

(84) Etats contractants désignés:
**AT BE CH DE ES FR GB GR IT LI LU NL SE**

(71) Demandeur: **SYNTHELABO**
**58 rue de la Glacière**
**F-75013 Paris (FR)**

(72) Inventeur: **George, Pascal**
**39, rue Henri de Vilmorin**
**F-94400 Vitry S/Seine (FR)**

**Allen, John**
**3, Square Lebrun**
**F-78180 Voisins Le Bretonneux (FR)**

(74) Mandataire: **Thouret-Lemaitre, Elisabeth et al**
**Service Brevets - SYNTHELABO 58, rue de la Glacière**
**F-75621 Paris Cédex 13 (FR)**

(54) **Imidazopyridines, leur préparation et leur application en thérapeutique.**

(57) Imidazopyridines répondant à la formule (I)

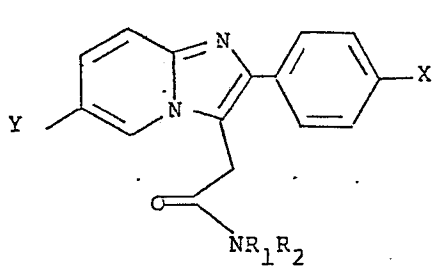

(I)

dans laquelle
soit X est $CH_3$ et Y est $CH_2OR$,
soit X est $CH_2OR$ et Y est $CH_3$,
R étant un radical $(C_{1-6})$alkyle,
$R_1$ est un radical $(C_{1-3})$alkyle,
$R_2$ est un radical $(C_{1-3})$alkyle.
Application en thérapeutique.

EP 0 267 111 A1

**Description**

IMIDAZOPYRIDINES, LEUR PREPARATION ET LEUR APPLICATION EN THERAPEUTIQUE

La présente invention a pour objet des imidazopyridines, leur préparation et leur application en thérapeutique.

Les composés de l'invention répondent à la formule (I) donnée en annexe 1,
dans laquelle
soit X est $CH_3$ et Y est $CH_2OR$,
soit X est $CH_2OR$ et Y est $CH_3$,
R étant un radical $(C_{1-6})$alkyle,
$R_1$ est un radical $(C_{1-3})$alkyle, $R_2$ est un radical $(C_{1-3})$alkyle.

Les composés de l'invention peuvent être préparés selon les schémas réactionnels donnés en annexes 1 et 2.

Selon le schéma réactionnel 1, on réduit une imidazo[1,2-a]pyridine de formule (III) au moyen d'hydrure double de lithium et d'aluminium en alcool correspondant (IV) que l'on alkyle au moyen d'un halogénure d'alkyle en présence d'une base telle que l'hydrure de sodium en éther (V). On introduit la chane acétamide à l'aide de l'amide N,N-dialkyl($R_1$, $R_2$)-glyoxylique puis on traite le composé (VI) avec du chlorure de thionyle et réduit le composé obtenu en imidazo[1,2-a]pyridine de formule (I).

Selon le schéma réactionnel 2, on part d'une imidazo[1,2-a]pyridine (II) à laquelle on fait subir le même traitement que dans le schéma 1.

Les imidazo[1,2-a]pyridines (III) et (II) sont préparées selon les schémas réactionnels donnés en annexe 3.

Les exemples suivants illustrent l'invention. Les analyses et les spectres IR et RMN confirment la structure des composés.

Exemple 1. N,N-diméthyl (méthyl-4 phényl)-2 méthoxyméthyl-6 imidazo[1,2-a]pyridine-3-acétamide (schéma réactionnel 1).

1. On introduit dans un ballon maintenu sous argon, 6 g (17,7 mmoles) de [(méthyl-4 phényl)-2 imidazo[1,2-a]pyridinyl]-6-carboxylate d'éthyle (III) (composé décrit dans le brevet français 85.06918), 1 g (26,5 mmoles) d'hydrure double de lithium et d'aluminium et 180 ml de tétrahydrofuranne. On maintient l'agitation à 0°C pendant 0,5 heure puis on hydrolyse le mélange. On extrait l'alcool (IV) au $CH_2Cl_2$ et le purifie par chromatographie sur colonne.
F = 183-186°C.

2. Dans un mélange de 35 ml de THF et 3,5 ml de DMF, on introduit 1,7 g (7,13 mmoles) de composé (IV), 684 mg (14,26 mmoles) de NaH à 50 % dans l'huile et 2 g (14,26 mmoles) d'iodure de méthyle. On agite le mélange à froid 40 mn, puis on ajoute 5 ml de méthanol et évapore à sec. On reprend le résidu à l'eau. On extrait l'éther (V) au $CH_2Cl_2$. Après séchage, filtration et évaporation du solvant on obtient un solide beige que l'on purifie par chromatographie sur colonne.
F = 113-114°C.

3. L'α-hydroxyacétamide (VI) est préparé de la façon suivante :

a) - on fait réagir 5,5 g (31,4 mmoles) de diéthylacétal du N,N-diméthylglyoxamide et 0,286 g (0,77 ml, 7,84 mmoles) d'acide chlorhydrique concentré dans 100 ml d'acide acétique, pendant 2 h, à 50°C.

b) - on traite 54 ml de la solution préparée ci-dessus par 1,27 g (15,45 mmoles) d'acétate de sodium pendant 1/4 h à 50°C, on y ajoute ensuite l'imidazopyridine (V) et laisse le mélange à cette température pendant 2 h. On évapore à sec le mélange, reprend le résidu par de l'eau et de l'ammoniaque diluée puis du $CH_2Cl_2$. La phase organique est décantée, séchée et le solvant évaporé : on purifie le résidu par chromatographie sur colonne de silice au moyen d'un mélange $CH_2Cl_2$ 97,5-méthanol 2,5.
L'α-hydroxyacétamide (VI) fond à 188-190°C.

4. La transformation du composé (VI) en composé (I) s'opère en deux étapes :

a) - on dissout 0,9 g (2,54 mmole) d'α-hydroxyacétamide (VII) dans 50 ml de $CH_2Cl_2$, y ajoute goutte à goutte 5 ml de $SOCl_2$ et laisse agiter 20 h à la température ambiante. On évapore le solvant et reprend le résidu à l'éther, puis le filtre et le sèche.

b) - on dissout 0,96 g (2,37 mmoles) du produit obtenu en 4.a. dans 70 ml de $CH_2Cl_2$, ajoute 1,1 g de Rongalite et laisse agiter à la température ambiante 3 h. On filtre l'insoluble, lave le filtrat à l'eau bicarbonatée puis à l'eau jusqu'à pH neutre. La phase organique est séchée puis le solvant évaporé. Le résidu d'évaporation est purifié par chromatograhie sur colonne de silice avec un mélange $CH_2Cl_2$-méthanol 98/2.
Le composé (I), N,N-diméthyl (méthyl-4 phényl)-2 méthoxyméthyl-6 imidazo[1,2-a]pyridine-3-acétamide, fond à 105-106°C.

Exemple 2. N,N,6-triméthyl (méthoxyméthyl-4 phényl)-2 imidazo[1,2-a]pyridine-3-acétamide. (Schéma réactionnel 2).

1. A 5,3 g ($1,9.10^{-2}$m) de (méthyl-6 imidazo[1,2-a]pyridinyl-2)-4 benzoate d'éthyle (II) (préparé à partir de (bromo-2 acétyl)-4 benzoate d'éthyle et de méthyl-5 pyridinamine-2 (voir annexe 3)) en suspension

dans 190 ml de tétrahydrofuranne (THF) sec et refroidis au bain de glace, on ajoute sous atmosphère d'argon 1,07 g (2,8.10−2 m, 1,5 eq) de $LiAlH_4$. On laisse agiter 3/4 h à cette température, puis hydrolyse, filtre sur célite, lave le précipité plusieurs fois par un mélange $CH_2Cl_2$-MeOH/1-1, puis évapore à sec. Ce résidu est purifié par chromatographie et cristallisé dans de l'éther.
Rendement = 57 %, F = 238-239° C.

2. A une suspension de 2,47 g (1,03.10−2 m) de méthyl-6 (hydroxyméthyl-4 phényl)-2 imidazo[1,2-a]pyridine (VII) dans un mélange de 50 ml de THF et de 5 ml de diméthylformamide, on ajoute sous atmosphère d'argon, 1 g (2,07 m, 2 eq) d'hydrure de sodium à 50 % dans de l'huile puis 1,3 ml (2,07 m, 2 eq) d'iodométhane. On laisse agiter 15 h à la température ambiante, puis hydrolyse l'excès d'hydrure par 10 ml de méthanol, évapore à sec, reprend entre l'eau et le chlorure de méthylène, lave, sèche, évapore. Le résidu est purifié par chromatographie, cristallisé dans de l'éther, filtré puis séché.
Rendement = 85 %, F = 158-160° C.

3. A 4,4 g (2,4.10−2 m) de diéthoxy-2,2 N,N-diméthylacétamide dissous dans 90 ml d'acide acétique, et dans un bain à 50° C, on ajoute goutte à goutte 0,58 ml (0,25 eq) d'HCl concentré et laisse agiter 2 h à cette température. Puis on ajoute 2 g (2,4.10−2 m) d'acétate de sodium, laisse 1/4 h et ajoute enfin 2,2 g (8,7.10−3 m) de méthyl-6 (méthoxyméthyl-4 phényl)-2 imidazo[1,2-a]pyridine (VIII) ; on continue le chauffage pendant 2 h, puis évapore à sec, reprend entre $CH_2Cl_2$ et $H_2O$, lave la phase organique à l'ammoniaque diluée, puis à l'eau jusqu'à pH neutre, sèche et évapore. L'huile résiduelle est purifiée par chromatographie, cristallisée dans de l'éther et séchée.
Rendement = 52 %, F = 152,5 - 153,5° C.

4. A 1,5 g (4,2.10−3 m) de α-hydroxy N,N-diméthyl méthyl-6 (méthoxyméthyl-4 phényl)-2 imidazo[1,2-a]pyridine-3-acétamide (IX) en solution dans 85 ml de chlorure de méthylène sec, on ajoute, goutte à goutte, 8,5 ml de chlorure de thionyle. On laisse agiter 15 h à la température ambiante, évapore à sec, reprend au pentane, cristallise et sèche sous vide. On obtient un solide blanc que l'on dissout dans 120 ml de chlorure de méthylène, ajoute 1,9 g (1,2.10−2 m, 3 eq) de Rongalite et laisse agiter 4 h à la température ambiante. Puis on filtre, lave le filtrat à l'eau bicarbonatée, puis à l'eau jusqu'à pH neutre, sèche et évapore. On purifie par chromatographie et fait cristalliser dans de l'éther.
Rendement = 75 %, F = 148-149° C.

Les composés de l'invention ont été soumis aux essais pharmacologiques suivants.

L'activité sédative ou hypnotique a été déterminée en observant l'action des composés sur l'ECoG du rat curarisé (Depoortere H., Rev. E.E.G. Neurophysiol., (1980) 10, 3, 207-214). Chez le rat curarisé, les produits à étudier sont injectés par voie intrapéritonéale ou orale aux doses croissantes de 1 à 30 mg/kg. Ils induisent des tracés de sommeil aux doses égales ou supérieures à 0,3 mg/kg i.p.

L'activité anticonvulsivante des composés a été déterminée selon le test de l'inhibition des convulsions cloniques induites par le pentétrazol chez la souris selon la méthode de Worms et coll (J. Pharmacol. Exp. Ther., 220 : 660-671). Chez des souris mâles (20-22 g) CD1 Charles River, les convulsions cloniques sont induites par injection i.v. de 35 mg/kg de pentétrazol, 30 mn après l'injection i.p. du produit à tester.

La DA 50 est la dose qui protège 50 % des animaux des convulsions cloniques provoquées par le pentétrazol. La DA 50 des composés de l'invention va de 0,1 à 10 mg/kg.

Les résultats des essais pharmacologiques montrent que les composés de l'invention sont actifs dans le domaine du système nerveux central et possèdent des propriétés anxiolytiques, inductrices de sommeil, hypnotiques et anticonvulsivantes ; les composés de l'invention sont utiles pour le traitement des états d'anxiétés, des troubles du sommeil et autres affections neurologiques et psychiatriques.

Les composés de l'invention peuvent être présentés sous toute forme appropriée pour l'administration par voie orale, ou parentérale, par exemple sous forme de comprimés, de dragées, de gélules, de solutions buvables ou injectables etc.. avec tout excipient approprié.

La posologie quotidienne peut aller de 0,5 à 2000 mg.

Annexe 1

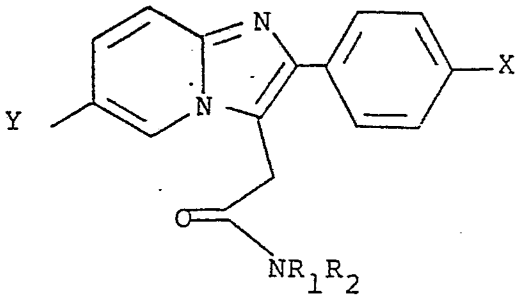

(I)

Schéma 1

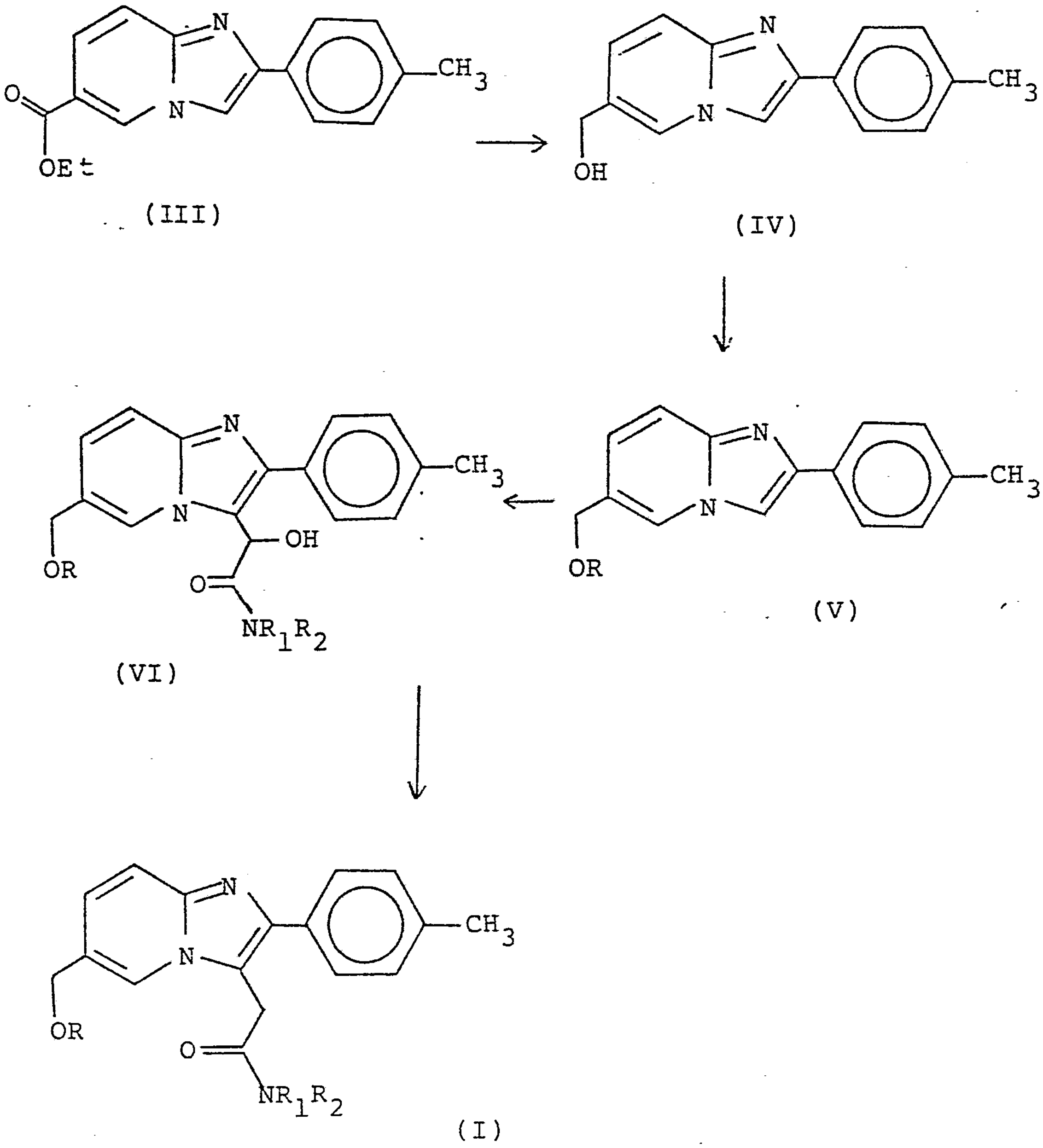

(III)

(IV)

(V)

(VI)

(I)

Annexe 2

Schéma 2

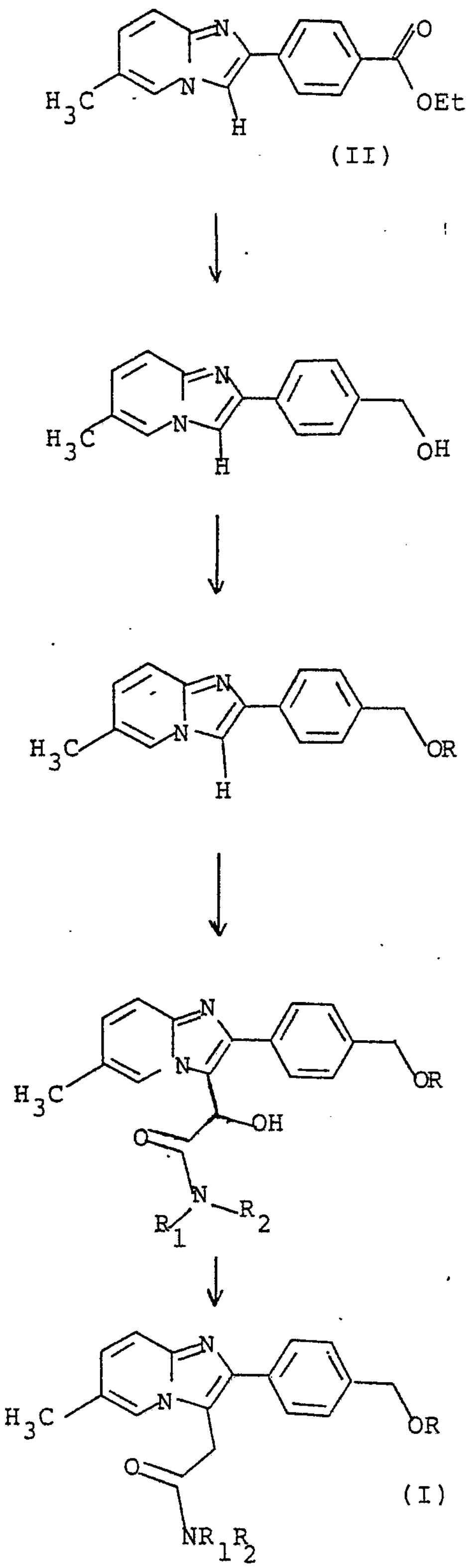

(II)

(VII)

(VIII)

(IX)

(I)

Annexe 3

Schéma 3

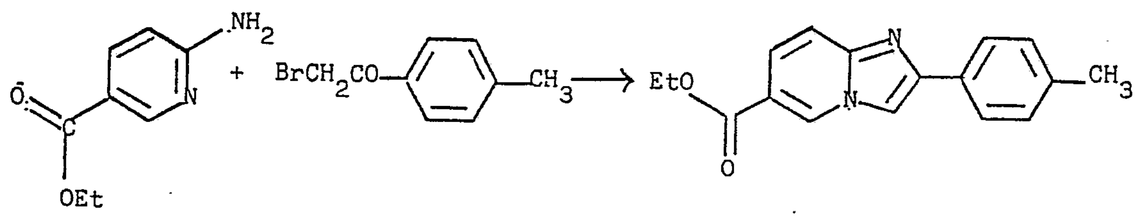

(III)

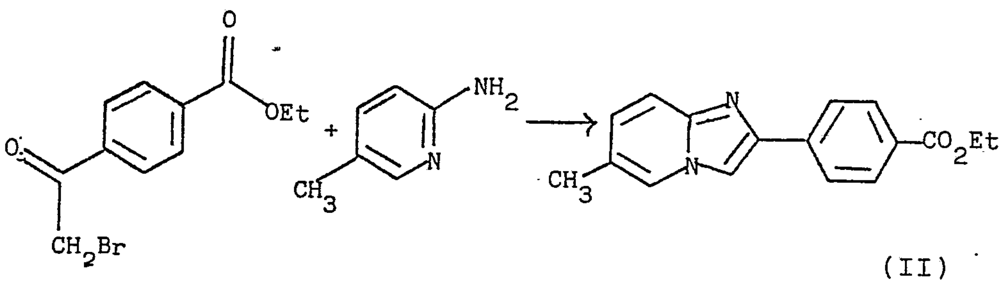

(II)

**Revendications**

1. Imidazopyridines répondant à la formule (I)

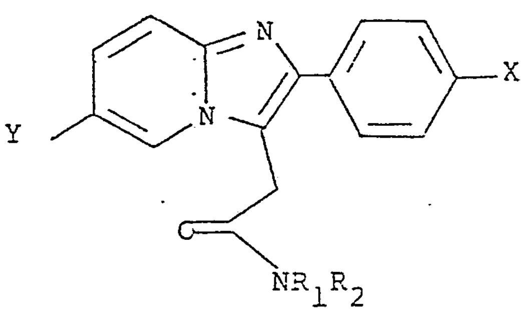

(I)

dans laquelle
soit X est $CH_3$ et Y est $CH_2OR$,
soit X est $CH_2OR$ et Y est $CH_3$,
R étant un radical $(C_{1-6})$alkyle,
$R_1$ est un radical $(C_{1-3})$alkyle,
$R_2$ est un radical $(C_{1-3})$alkyle.

2. Procédé de préparation des composés selon la revendication 1, dans lesquels X est $CH_3$ et Y est

$CH_2OR$, procédé caractérisé en ce que l'on réduit une imidazo[1,2-a]pyridine de formule (III)

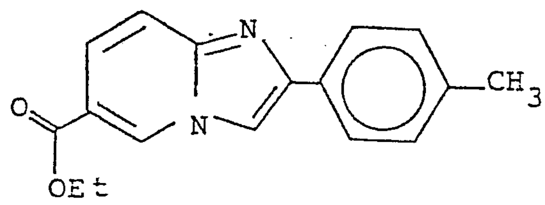

(III)

au moyen d'hydrure double de lithium et d'aluminium en alcool correspondant (IV)

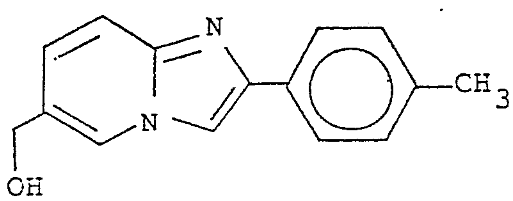

(IV)

que l'on alkyle au moyen d'un halogénure d'alkyle, en présence d'une base telle que l'hydrure de sodium, en éther (V),

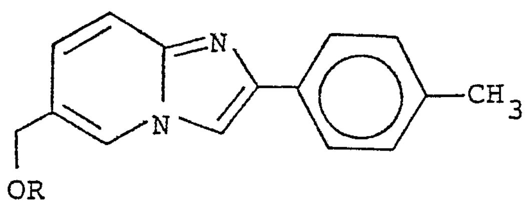

(V)

puis on introduit la chaîne acétamide à l'aide de l'amide N,N-dialkyl($R_1$, $R_2$)-glyoxylique et enfin on traite le composé (VI)

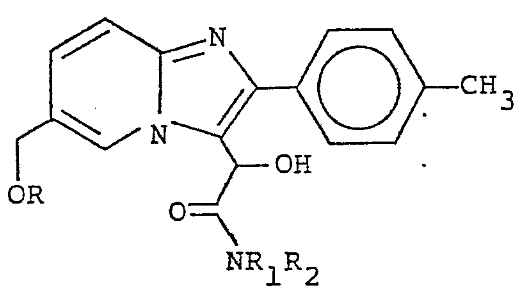

(VI)

avec du chlorure de thionyle puis réduit le composé obtenu en composé (I).

3. Procédé de préparation des composés selon la revendication 1, dans lesquels Y est $CH_3$ et X est $CH_2OR$, procédé caractérisé en ce que l'on réduit une imidazo[1,2-a]pyridine de formule (II)

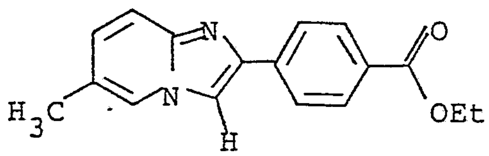

(II)

au moyen d'hydrure double de lithium et d'aluminium en alcool correspondant

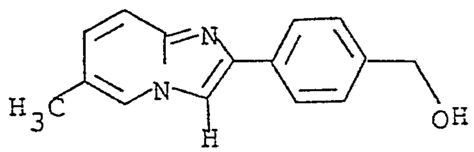

(VII)

que l'on alkyle au moyen d'un halogénure d'alkyle R Hal, en présence d'une base telle que l'hydrure de sodium, en éther,

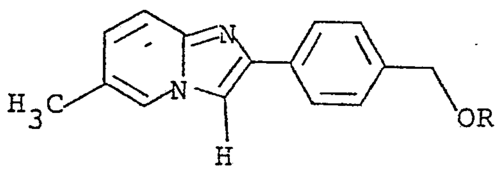

(VIII)

puis on introduit la chaîne acétamide à l'aide de l'amide N,N-dialkyl($R_1$, $R_2$)-glyoxylique et enfin on traite le composé

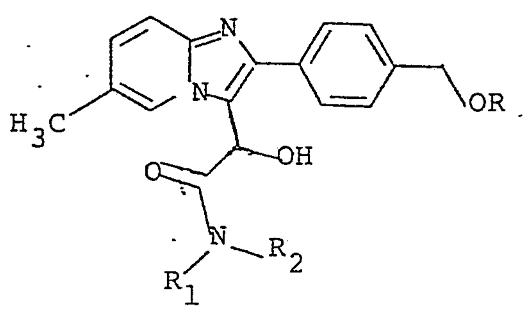

(IX)

avec du chlorure de thionyle puis réduit le composé obtenu en composé (I).

4. Médicament caractérisé en ce qu'il contient un composé selon la revendication 1.

5. Composition pharmaceutique caractérisée en ce qu'elle contient un composé selon la revendication 1 en association avec tout excipient approprié.

Revendications pour les Etats contractants suivants : ES et GR.

1. Procédé de préparation des imidazopyridines répondant à la formule (I)

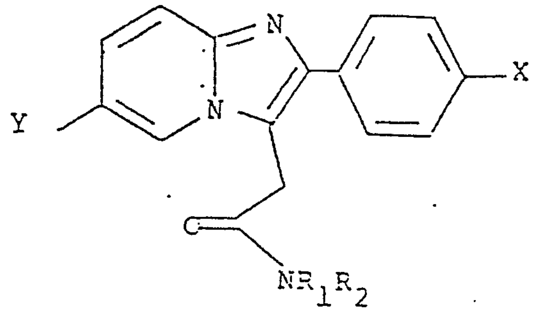

(I)

dans laquelle
X est $CH_3$ et Y est $CH_2OR$,
R étant un radical $(C_{1-6})$alkyle,
$R_1$ est un radical $(C_{1-3})$alkyle,
$R_2$ est un radical $(C_{1-3})$alkyle,
procédé caractérisé en ce que l'on réduit une imidazo[1,2-a]pyridine de formule (III)

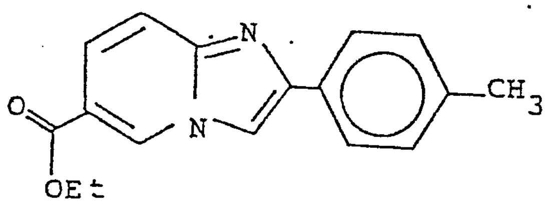

(III)

au moyen d'hydrure double de lithium et d'aluminium en alcool correspondant (IV)

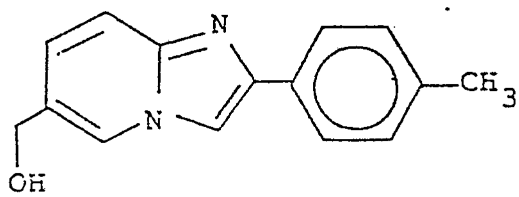

(IV)

que l'on alkyle au moyen d'un halogénure d'alkyle, en présence d'une base telle que l'hydrure de sodium, en éther (V),

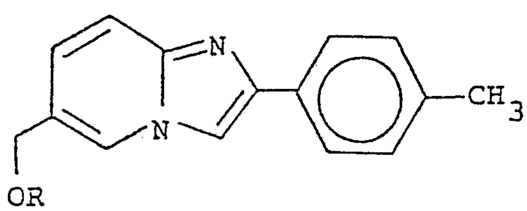

(V)

puis on introduit la chaîne acétamide à l'aide de l'amide N,N-dialkyl($R_1$, $R_2$)-glyoxylique et enfin on traite le composé (VI)

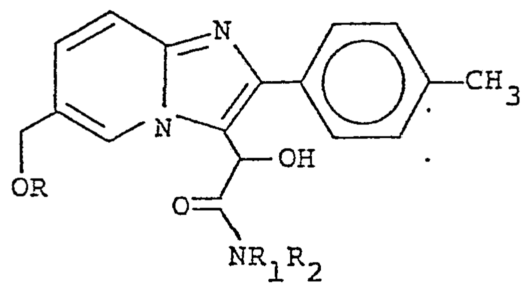

(VI)

avec du chlorure de thionyle puis réduit le composé obtenu en composé (I).

2. Procédé de préparation des imidazopyridines répondant à la formule (I)

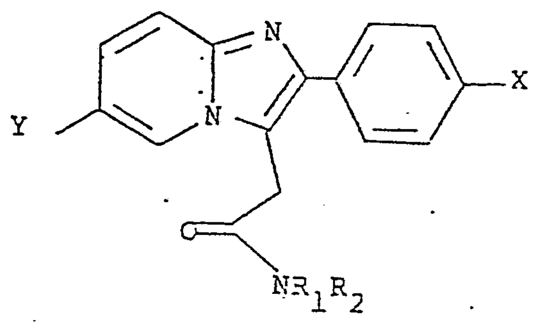

(I)

dans laquelle
X est $CH_2OR$ et Y est $CH_3$,
R étant un radical $(C_{1-6})$alkyle,
$R_1$ est un radical $(C_{1-3})$alkyle,
$R_2$ est un radical $(C_{1-3})$alkyle,
procédé caractérisé en ce que l'on réduit une imidazo[1,2-a]pyridine de formule (II)

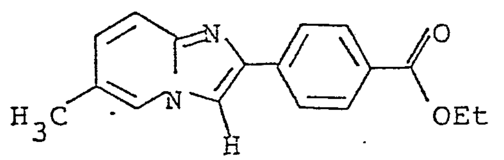

(II)

au moyen d'hydrure double de lithium et d'aluminium en alcool correspondant

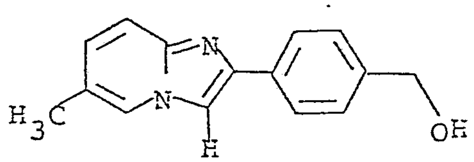

(VII)

que l'on alkyle au moyen d'un halogénure d'alkyle R Hal, en présence d'une base telle que l'hydrure de sodium, en éther,

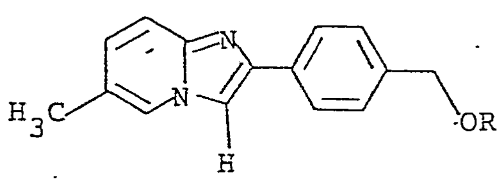

(VIII)

puis on introduit la chaîne acétamide à l'aide de l'amide N,N-dialkyl($R_1$, $R_2$)-glyoxylique et enfin on traite le composé

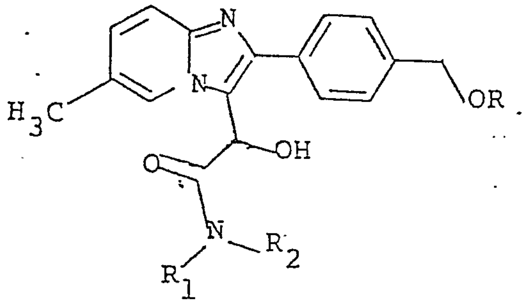

(IX)

avec du chlorure de thionyle puis réduit le composé obtenu en composé (I).

Office européen des brevets

# RAPPORT DE RECHERCHE EUROPEENNE

Numero de la demande

EP 87 40 2463

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 050 563 (SYNTHELABO)<br>* Revendications 1,9-10; page 13, lignes 29-34; page 14, lignes 5-9 *<br>--- | 1,4-5 | C 07 D 471/04<br>A 61 K 31/435//<br>(C 07 D 471/04<br>C 07 D 235:00<br>C 07 D 221:00 ) |
| A | EP-A-0 092 459 (SYNTHELABO)<br>* Revendications 1,6-7; page 14, lignes 12-17,24-28 *<br>--- | 1,4-5 | |
| A | EP-A-0 172 097 (SYNTHELABO)<br>* Revendications 1,5,6; page 14, lignes 21-26,34-38 *<br>--- | 1,4-5 | |
| P,A | FR-A-2 581 646 (SYNTHELABO)<br>* Revendications, 1,4-5; page 8, lignes 24-28,33-37 *<br>----- | 1,4-5 | |
| | | | **DOMAINES TECHNIQUES RECHERCHES (Int. Cl.4)** |
| | | | C 07 D 471/00<br>A 61 K 31/00 |

Le présent rapport a été établi pour toutes les revendications

| Lieu de la recherche | Date d'achèvement de la recherche | Examinateur |
|---|---|---|
| LA HAYE | 11-01-1988 | SCARPONI U. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)